# EUROPÄISCHE PATENTSCHRIFT

(11) **EP 0 670 692 B1**
(45) Veröffentlichungstag und Bekanntmachung des Hinweises auf die Patenterteilung: **22.10.1997**
(21) Anmeldenummer: 94901840.2
(22) Anmeldetag: 22.11.1993
(51) Int. Cl.: A61B 1/12, A61L 2/20

(54) **STERILISIEREINRICHTUNG FÜR ENDOSKOPKANÄLE**
DEVICE FOR STERILISING ENDOSCOPICAL CHANNELS
DISPOSITIF PERMETTANT DE STERILISER DES CANAUX ENDOSCOPIQUES

(30) Priorität: 24.11.1992 DE 4239414
(43) Veröffentlichungstag der Anmeldung: 13.09.1995
(73) Patentinhaber: MOLTRECHT, Wilfried, D-21335 Lüneburg (DE)
(72) Erfinder: MOLTRECHT, Wilfried, D-21335 Lüneburg (DE)
(74) Vertreter: Emmel, Thomas, Dipl.-Biol., Dr.
(86) Internationale Anmeldenummer: EP9303268
(87) Internationale Veröffentlichungsnummer: WO9412090

(56) Entgegenhaltungen:
- EP-A- 0 452 780
- DE-A- 3 722 116
- US-A- 4 337 223

## Beschreibung

Die Erfindung bezieht sich auf eine Sterilisiereinrichtung für Endoskopkanäle nach dem Oberbegriff des Anspruches 1.

Herkömmliche Endoskope, d.h. in den menschlichen Körper einführbare flexible oder starre, langgestreckte Geräte mit oder ohne Optik, besitzen ein oder mehrere beidseitig offene Kanäle, z.B. Spül- oder Arbeitskanäle, die während eines Eingriffes in das Operationsfeld münden. Um Infektionen vorzubeugen, ist es erforderlich, daß diese Kanäle vor jedem Eingriff sterilisiert werden.

Die Sterilisation derartiger Kanäle ist jedoch insbesondere bei den z.B. in der Angiologie verwendeten flexiblen Endoskopen schwierig. Derartige Endoskope enthalten nur begrenzt hitzebeständige Materialien, so daß Autoklavieren als Sterilisationsmethode ausscheidet. Weitere Möglichkeiten, wie z.B. die Acetylenoxydbegasung oder Gammabestrahlung setzen aufwendige zentrale Vorrichtungen voraus und sind daher nicht oder nur in Grenzen praktikabel.

Man ist daher dazu übergegangen, die Endoskope vor Ort mit Wasserstofperoxyd durchzuspülen. Dazu wird das eine Ende des Kanals über einen Adapter mit einem begrenzt verformbaren Vorratsgefäß verbunden, in dem eine zerbrechbare, Wasserstoffperoxyd enthaltene Ampulle angeordnet ist. Über sein anderes Ende wird der Kanal mit Vakuum beaufschlagt. Nachdem das Vakuum in dem Kanal aufgebaut ist, wird die Ampulle durch Eindrücken der Vorratsgefäßwände zerbrochen und das freigesetzte Wasserstoffperoxyd durch den Kanal gesaugt, wobei die Sterilisation erfolgt. Ein entsprechendes Verfahren ist z.B. in Hyg. Med. Volume 17, Nr. 12, 1992 auf Seiten 537 - 543 beschrieben.

Das Arbeiten mit dieser bekannten Sterilisiereinrichtung besitzt jedoch eine Reihe von Nachteilen. Ein Nachteil ist, daß die Zerstörung der Ampulle durch Eindrücken der Vorratsgefäßwände manchmal nur unter goßer Kraftaufwendung möglich ist, in einigen Fällen sogar überhaupt nicht gelingt. Ein weiterer Nachteil ist, daß gegebenenfalls kleine Bruchstücke der Ampulle in den Kanal gesaugt werden. Schließlich ist es fraglich, ob sich die Sterilisation mit den beschriebenen bekannten Vorratsgefäßen reproduzierbar durchführen läßt. Es ist nämlich erforderlich, daß die Freigabe des Sterilisationsmittels schlagartig durch eine relativ große Öffnung erfolgt, weil nur so eine ausreichende Vernebelung des Sterilisationsmittels in dem Kanal möglich ist. Ob sich eine derartige Öffnung der Ampulle in allen Fällen durch Eindrücken des Vorratsgefäßes erreichen läßt, ist fraglich.

Um diesem Problem zu begegnen, ist bereits angedacht worden, die Membran derart auszubilden, daß sie nach Anlegen des Vakuums selbsttätig aufreißt. Theoretisch wäre dies möglich, indem man die Membran in dem Bereich, der durch das Vakuum die stärkste Ausstülpung erfährt, mit einer Schwächungszone versieht. Derartige Membranen sind jedoch äußerst schwierig herzustellen. Probleme bereitet insbesondere die Tatsache, daß eine derartige Membran einerseits bei Vakuumbeaufschlagung garantiert zerreißen muß. Andererseits muß dieselbe Membran aber unter normalen Druckbedingungen einen gasdichten Einschluß des in dem Vorratsgefäß enthaltenen Wasserstoffperoxyds auch über längere Lagerzeiten gewährleisten. Beide Anforderungen lassen sich nur schwer miteinander vereinbaren, so daß diese Lösung zur Zeit kaum realisierbar ist.

In diesem Zusammenhang wird weiterhin auf die EP-A-0 452 780 verwiesen. Bei der hier offenbarten Vorrichtung wird der zu sterilisierende Raum über einen Adapter mit einem Vorratsgefäß verbunden, das Wasserstoffperoxyd enthält. In dem Adapter ist eine zerstörbar ausgebildete Membran und in dem Vorratsgefäß eine Nadel vorgesehen. Beim Einsetzen des Adapters in das Vorratsgefäß zerstört die Nadel die Membran und das Wasserstoffperoxyd wird freigesetzt. Diese Vorrichtung ist jedoch relativ aufwendig in der Bedienung.

Aufgabe der Erfindung ist es daher, eine Sterilisiereinrichtung zu schaffen, die einfach herstellbar ist und die im Zuge der Sterilisation problemlos und funktionssicher einsetzbar ist.

Gelöst wird diese Aufgabe mit einer Sterilisiereinrichtung, die die kennzeichnenden Merkmale des Anspruches 1 besitzt.

Das Prinzip der Lösung besteht darin, daß in der Sterilisiereinrichtung eine Nadel so angeordnet ist, daß deren Spitze von außen auf die Membran weist. Membran und Nadel sind relativ zueinander bewegbar in der Sterilisiereinrichtung ausgebildet, dergestalt, daß die Membran mit der Nadelspitze im Zuge der Relativbewegung punktiert wird und das eingeschlossene Sterilisiermittel, insbesondere Wasserstoffperoxyd, freigibt.

Die Vorteile der erfindungsgemäßen Sterilisiereinrichtung liegen auf der Hand. So stellt die in der Sterilisiereinrichtung angeordnete Nadel sicher, daß eine Zerstörung der Membran und die damit bezweckte Freisetzung des Sterilisiermittels problemlos und zuverlässig erfolgt. Zum Einsatz können dabei nahezu alle herkömmlichen, gasdichten Membranen kommen. Je nach Membraneigenschaften können unterschiedliche Nadeln zur Punktion eingesetzt werden, wodurch in allen Fällen die gewünschte schnelle Freisetzung des Sterilisiermittels erfolgen kann.

Es ist z.B. denkbar, die Membran aus einem flexiblen, bei Punktion aufreißenden Material herzustellen. In einem derartigen Fall reicht zur Punktion eine einfache kompakte Nadel aus. Andererseits kann die Membran jedoch auch aus einem rißfest durchstechbarem Material hergestellt sein. Bei dieser Variante muß die Nadel dann in Form einer Hohlkanüle ausgebildet sein, durch die das Sterilisiermittel aus dem Vorratsgefäß in den Endoskopkanal entweichen kann. Auf die hier möglichen Varianten wird weiter unten noch einmal eingegangen.

Vorteilhafte Ausgestaltungen der Erfindung sind in den Ansprüchen 2 bis 10 geschützt.

Nach Anspruch 2 ist in vorteilhafter Weise vorgesehen, daß die Nadel ortsfest angeordnet und die Membran bewegbar ausgebildet ist. Die Spitze der Nadel ist dabei insbesondere auf einen zentralen Bereich der Membran ausgerichtet. Die beschriebene Ausgestaltung erlaubt eine besonders funktionssichere und einfache Verwirklichung.

So ist es nach Anspruch 3 z.B. möglich, das Vorratsgefäß und den Adapter ortsfest in einer definierten Position miteinander zu verbinden und die Membran in dem Vorratsgefäß derart auszubilden, daß sie bei Anlegen des Vakuums gegen die Spitze der Nadel gesaugt und dabei punktiert wird. Mit einer derartigen Sterilisiereinrichtung läßt sich die Sterilisierung des Endoskopkanales unter minimalem Bedienungsaufwand, fast automatisch, durchführen.

Dabei ist es gemäß Anspruch 4 möglich, die Membran aus einem bei Verletzung aufreißenden Material herzustellen. Bei Einsatz einer derartigen Membran ist auf alle Fälle sichergestellt, daß die Freisetzung des Sterilisiermittels schlagartig bei Punktierung der Membran mit der Nadelspitze erfolgt.

Derartige geeignete Membranen bestehen jedoch in der Regel aus einem anderen Material als das Vorratsgefäß und lassen sich daher nicht einstückig mit diesem herstellen. Daher sieht eine weitere Ausgestaltung gemäß Anspruch 5 vor, daß das Vorratsgefäß in Querrichtung aus zwei vakuumdicht miteinander zu verbindenden Teilen ausgebildet ist. Die aus geeignetem Material bestehende Membran kann dann in dem Verbindungsbereich zwischen den beiden Teilen mit ihrem Rand eingespannt werden. So kann auf vorteilhafte Weise die Wahl des Membranmaterials unabhängig von der Wahl des Vorratsgefäßmateriales erfolgen.

Bei der eben beschriebenen Ausgestaltung wird die Nadel in der Regel in dem äußeren Teil des Vorratsgefäßes angeordnet sein.

Genauso gut ist es aber möglich, die Nadel gemäß Anspruch 6 in dem Adapter auszubilden. Eine derartige Ausgestaltung besitzt eine Reihe von Vorteilen. Hauptvorteil ist, daß der Adapter in der Regel wiederverwendet wird, während das Vorratsgefäß ein Wegwerfteil ist. Da die Ausbildung der Nadel einen relativ aufwendigen Herstellungsprozeß bedingt, kann man auf diese Weise die Herstellungskosten beträchtlich minimieren. Ein weiterer Vorteil der Trennung von Nadel und Vorratsgefäß besteht darin, daß eine unbeabsichtigte Durchstechung der Membran (z.B. bei Druckschwankungen - Transport im Flugzeug) verhindert wird.

Ein weiterer erheblicher Vorteil besteht darin, daß die Membran bei dieser Ausgestaltung nun nicht mehr im Inneren des Vorratsgefäßes angeordnet werden muß. Es ist vielmehr in einer weiteren vorteilhaften Ausgestaltung gemäß Anspruch 7 möglich, die Membran auf dem Rand des Vorratsgefäßes durch Verschweißen oder Verkleben zu befestigen, was eine beträchtliche konstruktive Vereinfachung des Vorratsgefäßes darstellt.

Die bislang besprochenen Ausgestaltungen betreffen in erster Linie eine Sterilisiereinrichtung, bei der die Bewegung der Membran gegenüber der Nadelspitze aufgrund des angelegten Vakuums erfolgt.

Die Bewegung der Membran gegenüber der Nadel kann jedoch auch auf andere Weise erfolgen. So ist es nach Anspruch 8 möglich, Vorratsgefäß und Adapter dergestalt auszubilden, daß das Vorratsgefäß vakuumdicht in dem Adapter verschiebbar ist. Ordnet man nun die Nadel in dem Adapter und die Membran an geeigneter Stelle in dem Vorratsgefäß an, so ist es möglich, die Membran durch einfaches Einschieben des Vorratsgefäßes in den Adapter zu punktieren.

Besonders vorteilhaft an dieser Ausgestaltung ist, daß man in der Wahl des Membranmaterials nahezu keinen Beschränkungen unterliegt. Da sich die Membran nicht mehr ausstülpen muß, kann sie zwar nach wie vor aus flexiblem, aber auch aus unflexiblem Material bestehen. Weitere in diesem Zusammenhang vorteilhafte Möglichkeiten ergeben sich, wenn man als Nadel eine durchgehende Hohlkanüle, wie in Anspruch 9 vorgeschlagen, vorsieht. In diesem Fall kann gemäß Anspruch 10 die Membran auch aus einem rißfest punktierbaren Material bestehen. Hier ist es also gegebenenfalls sogar möglich, die Membran aus dem gleichen Material wie das Vorratsgefäß herzustellen. Über den von der Hohlkanüle geschaffenen Durchgang durch die Membran kann das Sterilisiermittel bei angelegtem Vakuum ebenso gut entweichen, wie dies bei einer aufreißenden Membran der Fall ist.

Im folgenden soll die Erfindung anhand mehrerer, unterschiedliche Ausführungsbeispiele zeigenden Abbildungen näher erläutert werden. Dabei zeigt
- Fig. 1: in einer Übersichtsdarstellung einen Endoskopkanal mit aufgesetzter Sterilisiereinrichtung,
- Fig. 2: in einer Ausschnittsvergrößerung den die Membran tragenden Bereich des Vorratsgefäßes vor Kontakt mit der Nadel,
- Fig. 3: den in Fig. 2 gezeigten Bereich nach Kontakt der Membran mit der Nadel,
- Fig. 4: in einer Ausschnittsvergrößerung die Anordnung einer Membran in einem zweiteiligen Vorratsgefäß,
- Fig. 5: eine weitere Möglichkeit, die Nadel zu haltern,
- Fig. 6: ein Ausführungsbeispiel, bei dem die Nadel als Hohlkanüle ausgebildet ist,
- Fig. 7: ein Ausführungsbeispiel, bei dem die Nadel in dem Adapter angeordnet ist,
- Fig. 8: wie Fig. 7 ein Ausführungsbeispiel, bei dem die Nadel in dem Adapter angeordnet ist, jedoch mit dem Unterschied, daß die Membran im Inneren des Vorratsgefäßes ausgebildet ist und
- Fig. 9: schließlich ein weiteres Ausführungsbeispiel, bei dem das Vorratsgefäß über einen ersten Anschlag hinweg in den Adapter vorschiebbar ausgebildet ist.

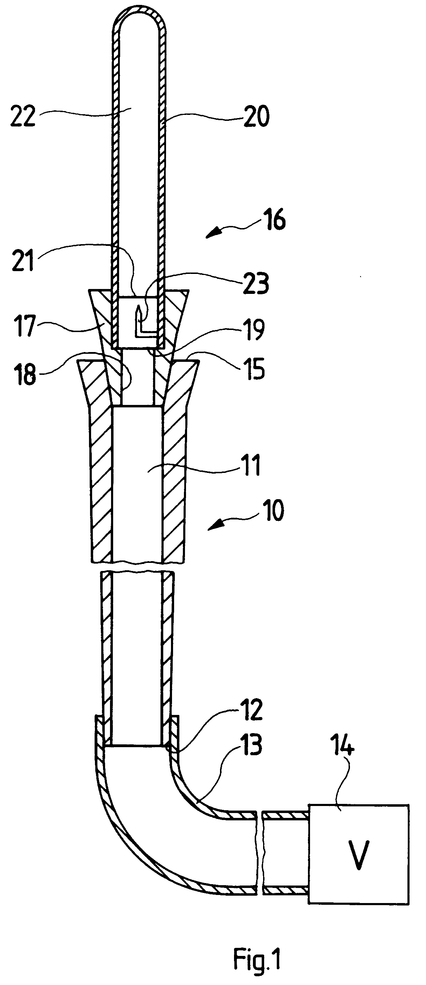

In Fig. 1 erkennt man schematisch dargestellt ein Endoskop 10, in dem ein Kanal 11 ausgebildet ist. Der Kanal 11 steht über einen an einem Ende 12 des Endoskopes 10 angeordneten Schlauch 13 mit einer Vakuumpumpe 14 in Verbindung. Am anderen Ende 15 des Endoskopes 10 ist ein Ausführungsbeispiel der erfindungsgemäßen Sterilisiereinrichtung 16 angeordnet. Die Sterilisiereinrichtung 16 besteht aus einem Adapter 17, der in den Kanal 11 vakuumdicht eingesetzt ist. Der Adapter 17 besitzt eine Durchbohrung 18, deren unteres Ende in den Kanal 11 mündet und in deren oberen Endbereich ein mit einer Öffnung 19 versehenes Vorratsgefäß dergestalt eingesetzt ist, daß die Öffnung 19 zum Kanal 11 weist. Das Vorratsgefäß 20 sowie der Adapter 17 sind derart ausgebildet, daß eine vakuumdichte Verbindung zwischen dem Kanal 11 des Endoskopes 10 und dem Vorratsgefäß 20 gegeben ist.

Im Vorratsgefäß 20 ist eine Membran 21 angeordnet, die einen abgeschlossenen Bereich 22 in dem Gefäß 20 begrenzt. In diesem abgeschlossenen Bereich 22 befindet sich das Sterilisiermittel.

Als Sterilisiermittel besonders geeignet ist Wasserstoffperoxyd. In Frage käme jedoch z.B. auch Äthylendioxyd. Geeignet sind grundsätzlich alle flüssigen bzw. verflüssigbaren Sterilisiermittel, die bei schlagartiger Vakuumbeaufschlagung zerstäub- bzw. vergasbar sind.

In dem Vorratsgefäß ist eine Nadel 23 angeordnet, deren Spitze von außen auf die Membran 21 weist.

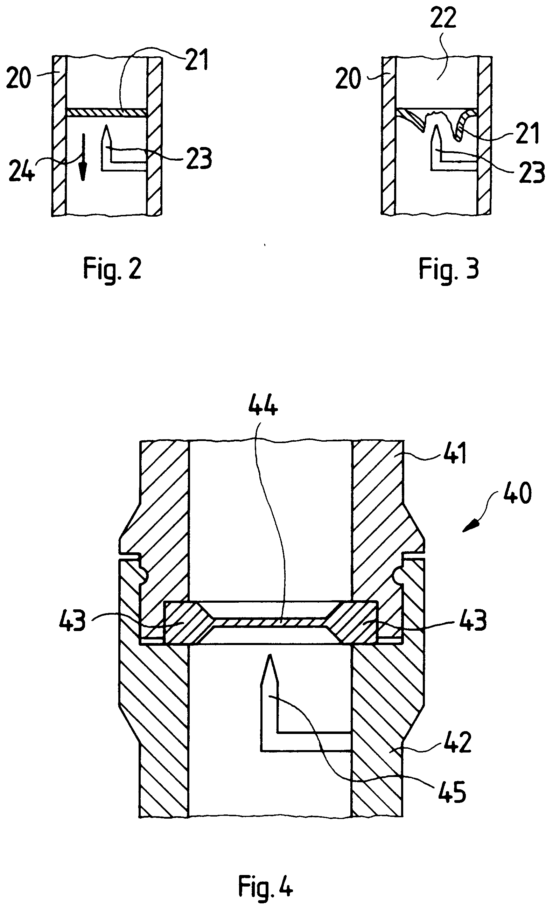

In dem in Fig. 1 gezeigten Zustand ist kein Vakuum an den Kanal 11 angelegt. Was bei Anlegen von Vakuum passiert, zeigen im Ausschnitt die Fig. 2 und 3.

In Fig. 2 erkennt man im Ausschnitt den die Membran 21 tragenden Bereich des Vorratsgefäßes 20 und die unterhalb der Membran angeordnete Nadel 23. Wird nun Vakuum angelegt, dann stülpt sich die aus einem flexiblen Material bestehende Membran in Richtung des Pfeiles 24 aus, gelangt in Eingriff mit der Spitze der Nadel 23 und zerreißt, wie in Fig. 3 dargestellt.

Nach dem Zerreißen der Membran wird das in dem bislang abgeschlossenen Bereich 22 enthaltene Sterilisiermittel schlagartig vergast bzw. zerstäubt und in dieser Form in den Kanal 11 des Endoskopes 10 hineingesaugt. Auf diese Weise ist ein optimaler Kontakt zwischen den Wänden des Kanales 11 und dem Sterilisiermittel möglich.

Fig. 4 zeigt in einem weiteren Ausführungsbeispiel ein Vorratsgefäß 40. Man erkennt, daß das Vorratsgefäß 40 aus einem inneren Teil 41 und einem äußeren Teil 42 besteht. Zwischen den Teilen 41 und 42 ist eine mit verdicktem Rand 43 ausgebildete Membran 44 eingespannt. Der äußere Teil 42 trägt eine Nadel 45, deren Spitze auf die Membran 44 gerichtet ist.

Die Anordnung der Membran in der gezeigten Weise stellt eine besonders einfache Möglichkeit dar, Vorratsgefäß und Membran aus unterschiedlichen Materialien herzustellen.

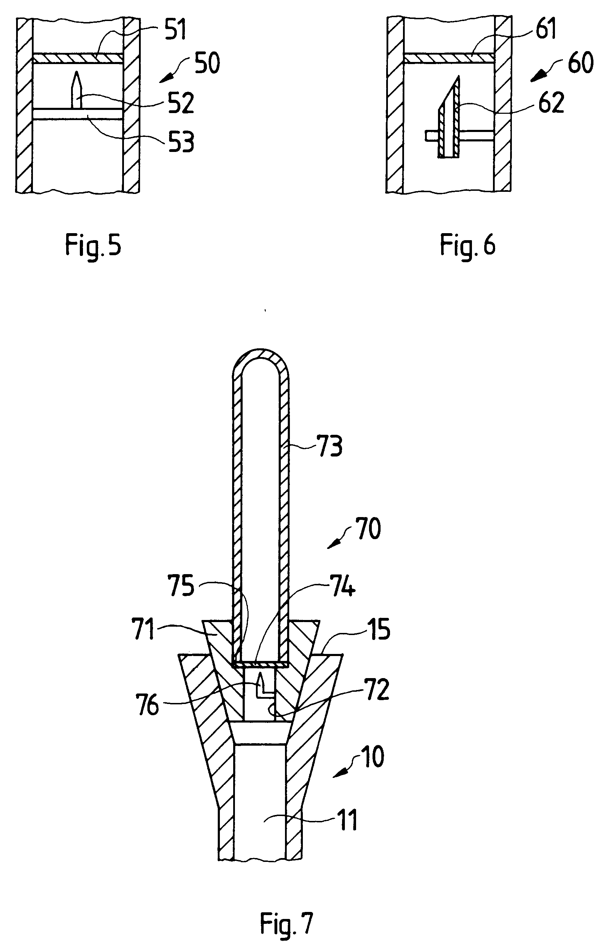

Die Fig. 5 und 6 zeigen in weiteren Ausführungsbeispielen Vorratsgefäße 50 bzw. 60. In beiden Fällen sind die Vorratsgefäße aus Übersichtlichkeitsgründen wieder einteilig gezeichnet. Hier ist es aber selbstverständlich auch möglich, die gezeigten Membranen 51 und 61, wie in Fig. 4 gezeigt, zwischen zwei separaten Vorratsgefäßteilen einzuspannen.

Die Fig. 5 und 6 zeigen unterschiedliche Möglichkeiten zur Anordnung bzw. Ausbildung der Nadel. So erkennt man in Fig. 5 eine Nadel 52, die von einem sich quer durch das Vorratsgefäß erstreckenden Steg 53 getragen wird. Diese Art der Befestigung der Nadel kann stabiler sein als die in den Fig. 1-4 gezeigte einarmige Befestigung.

Fig. 6 zeigt eine Nadel 62, die als Hohlkanüle ausgebildet ist. Der Vorteil einer derartigen Nadel besteht darin, daß im Zweifelsfall nach Durchstechen der Membran 61 das Sterilisiermittel auch durch die Hohlnadel 62 entweichen kann. Dies ist insbesondere dann von Vorteil, wenn die Membran nicht in der gewünschten Weise aufreißt.

Fig. 7 zeigt in einem weiteren Ausführungsbeispiel eine Sterilisiereinrichtung 70, die von den eben gezeigten Ausführungsbeispielen grundsätzlich abweicht. Man erkennt wieder das Endoskop 10 mit dem Kanal 11. Am proximalen Ende 15 des Endoskopes 10 ist ein Adapter 71 in den Kanal 11 eingesetzt. Der Adapter 71 besitzt eine Durchbohrung 72, die in den Kanal 11 mündet. In den oberen Endbereich der Durchbohrung 72 ist ein das Sterilisiermittel enthaltendes Vorratsgefäß 73 eingesetzt. Soweit bestehen keine Unterschiede zu den zuvor gezeigten Ausführungsbeispielen.

Ein wesentlicher Unterschied besteht jedoch darin, daß die Nadel 76 in der Durchbohrung 72 des Adapters 71 angeordnet ist. Die Nadel 76 weist mit ihrer Spitze auf eine Membran 74, die auf den Rand 75 des Vorratsgefäßes 73 aufgeschweißt ist. Auch diese Anordnung der Membran 74 am öffnungsseitigen Ende des Vorratsgefäßes 73 stellt einen Unterschied zu den bislang gezeigten Ausführungsbeispielen dar.

Wird Vakuum an den Kanal 11 angelegt, so wird die Membran 74 in der gewohnten Weise über die Spitze der Nadel 76 gezogen und dabei zerstört. Das in dem Vorratsgefäß 73 befindliche Sterilisiermittel kann in den Kanal 11 entweichen.

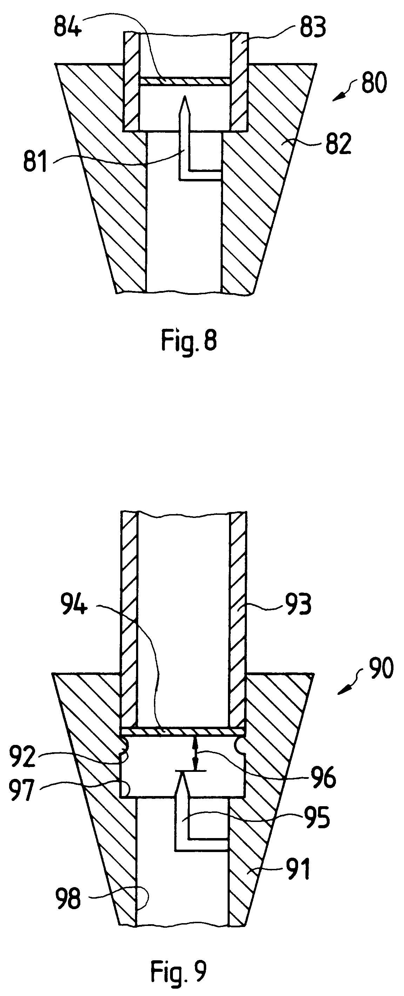

In Fig. 8 ist in einem weiteren Ausführungsbeispiel eine Sterilisiereinrichtung 80 gezeigt. Auch hier ist eine Nadel 81 in dem Adapter 82 angeordnet. Wesentlicher Unterschied zu Fig. 7 ist, daß hier ein Vorratsgefäß 83 vorgesehen ist, das im Inneren die Membran 84 trägt. Dieses Ausführungsbeispiel ist zwar gegenüber dem in Fig. 7 gezeigten möglicherweise schwieriger herzustellen (unter Umständen ist hier eine zweiteilige Ausbildung des Vorratsgefäßes erforderlich, wenn man auf bestimmte Membranmaterialien zurückgreifen möchte). Andererseits hat das in Fig. 8 gezeigte Ausführungsbeispiel den Vorteil, daß die Membran im Innern des Vorratsgefäßes 83 relativ gut geschützt ist und nicht ohne weiteres durch ungewolltes Einritzen etc. verletzt werden kann.

Fig. 9 zeigt in einem weiteren Ausführungsbeispiel eine Sterilisiereinrichtung 90, die gegenüber den bislang gezeigten Ausführungsbeispielen grundsätzlich anders arbeitet. Hier ist ein Adapter 91 mit einer Durchbohrung 98 vorgesehen, in der ein erster Anschlag 92 ausgebildet ist. Gegen diesen ersten Anschlag 92 ist ein Vorratsgefäß 93 in den Adapter 91 eingeschoben. Das Vorratsgefäß 93 ist öffnungsseitig mit einer Membran 94 verschlossen.

In dem Adapter 91 ist eine Nadel 95 angeordnet, deren Spitze in gewohnter Weise zur Membran 94 gerichtet ist.

In der gezeigten Einschubposition besteht ein Abstand 96 zwischen der Spitze der Nadel 95 und der Membran 94.

Der Anschlag 92 ist so beschaffen, daß sich das Vorratsgefäß bei geeigneter Kraftbeaufschlagung über diesen Anschlag tiefer in den Adapter 91 bis hin zu einem zweiten Anschlag 97 vorschieben läßt. Im Zuge dieses Vorschubes gelangt die Membran 94 in Eingriff mit der Spitze der Nadel 95 und wird zerstört.

In dem in Fig. 9 gezeigten Ausführungsbeispiel wird also die Zerstörung der Membran nicht mehr durch eine vakuumbedingte Ausstülpung der Membran bewirkt. Sie erfolgt vielmehr durch Verschiebung der Membran 94 insgesamt gegen die Nadel 95.

Es wird deutlich, daß bei diesem in Fig. 9 gezeigten Ausführungsbeispiel das Membranmaterial nicht mehr unbedingt flexibel ausgestaltet sein muß. Wählt man darüber hinaus als Nadel eine Hohlnadel, so reicht es sogar aus, wenn das Membranmaterial lediglich durchstechbar ist.

Andererseits ist in Verbindung mit dem in Fig. 9 gezeigten Ausführungsbeispiel auch eine vakuumbedingte Zerstörung der Membran denkbar. So könnte man den ersten Anschlag 92 in dem Adapter 91 weglassen. Das Röhrchen wäre nur so weit in die Durchbohrung 98 einzuführen, bis erfahrungsgemäß eine ausreichende Abdichtung gewährleistet ist. Dann wird das Vorratsgefäß 93 durch Anlegen des Vakuums bis gegen den Anschlag 97 in den Adapter 91 hineingesaugt. Diese Möglichkeit würde eine weitgehende Automatisierung des Sterilisationsvorgangs ermöglichen.

In den dargestellten Ausführungsbeispielen sind jeweils die Nadeln nur schematisch angedeutet. Diese können als Stahlnadeln ausgebildet sein, die beispielsweise über Kunststoffarme mit dem aus Kunststoff bestehenden Vorratsgefäß oder Adapter verbunden sind. Die Nadeln können aber auch aus geeignetem Kunststoff geeigneter Härte und Spitzenausbildung bestehen und gegebenenfalls sogar mit dem Vorratsbehälter oder Adapter einstückig ausgebildet sein.

## Patentansprüche

1. Sterilisiereinrichtung 16, 70, 80, 90 für einen mit Vakuum beaufschlagbaren Endoskopkanal, mit einem in ein freies Ende des Endoskopkanales vakuumdicht einsetzbaren Adapter 17, 71, 91, in dem eine sich von dem Endoskopkanal nach außen erstreckende Durchbohrung ausgebildet ist und mit einem Vorratsgefäß (20) in dem ein flüssiges, unter Vakuum vergas- oder zerstäubbares Sterilisiermittel enthalten ist und das einen, eine Öffnung (19) tragenden Endbereich besitzt, mit dem das Vorratsgefäß vakuumdicht in die Durchbohrung des Adapters einsetzbar ist, wobei in dem Vorratsgefäß eine zerstörbar ausgebildete Membran 21, 44, 51, 61, 74, 84, 94 vorgesehen ist, die das Sterilisiermittel in dem Vorratsgefäß einschließt, **dadurch gekennzeichnet**, daß die Sterilisiereinrichtung (16, 70, 80, 90) eine mit ihrer Spitze von außen auf die Membran (21, 44, 51, 61, 74, 84, 94) weisende Nadel (23, 45, 52, 62, 76, 81, 95) aufweist und daß die Membran (21, 44, 51, 61, 74, 84, 94) und die Nadel (23, 45, 52, 62, 76, 81, 95) relativ zueinander bewegbar ausgebildet sind dergestalt, daß im Zuge der Relativbewegung die Spitze der Nadel (23, 45, 52, 62, 76, 81, 95) in die Membran (21, 44, 51, 61, 74, 84, 94) einstechbar ist.

2. Sterilisiereinrichtung nach Anspruch 1, **dadurch gekennzeichnet**, daß die Nadel (23, 45, 52, 62, 76, 81, 95) ortsfest in der Sterilisiereinrichtung (16, 70, 80, 90) angeordnet ist und die Membran (21, 44, 51, 61, 74, 84, 94) gegenüber der Nadel (23, 45, 52, 62, 76, 81, 95) bewegbar ausgebildet ist, wobei die Spitze der Nadel (23, 45, 52, 62, 76, 81, 95) insbesondere auf einen zentralen Bereich der Membran (21, 44, 51, 61, 74, 84, 94) ausgerichtet ist.

3. Sterilisiereinrichtung nach einem der Ansprüche 1 oder 2, **dadurch gekennzeichnet**, daß das Vorratsgefäß (20, 40, 50, 60, 73, 83) und der Adapter (17, 71, 82) in einer definierten Position ortsfest miteinander verbindbar sind und die Membran (21, 44, 51, 61, 74, 84) aus flexiblem Material ausgebildet und derart in einem Abstand zur Spitze der Nadel (23, 45, 52, 62, 76, 81) angeordnet ist, daß sie nach Anlegen des Vakuums an den Endoskopkanal (11) über die Spitze der Nadel (23, 45, 52, 62, 76, 81) saugbar und dabei punktierbar ist.

4. Sterilisiereinrichtung nach einem der Ansprüche 1-3, **dadurch gekennzeichnet**, daß die Membran (21, 44, 51, 61, 74, 84, 94) aus bei Verletzung aufreißendem Material besteht.

5. Sterilisiereinrichtung nach einem der Ansprüche 1 bis 4, **dadurch gekennzeichnet**, daß das Vorratsgefäß (40) in Querrichtung zweigeteilt ausgebildet ist und die Membran (44) mit ihrem Rand (43) im Verbindungsbereich der beiden Teile (41, 42) befestigt ist.

6. Sterilisiereinrichtung nach einem der Ansprüche 1 bis 5, **dadurch gekennzeichnet**, daß der Adapter (71, 82, 91) die Nadel (76, 81, 95) trägt.

7. Sterilisiereinrichtung nach Anspruch 6, **dadurch gekennzeichnet**, daß die Membran (74, 94) auf dem Rand des Vorratsgefäßes (73, 93) angeordnet ist.

8. Sterilisiereinrichtung, bei der gemäß einem der Ansprüche 6 oder 7 die Nadel (95) in dem Adapter (91) ausgebildet ist, **dadurch gekennzeichnet**, daß der Adapter (91) und das Vorratsgefäß (93) zur vakuumdichten Verschiebung des Vorratsgefäßes (93) in dem Adapter (91) ausgebildet sind, und die Positionen von Membran (94) und Nadel (95) so gewählt sind, daß die Spitze der Nadel (95) während des Einschiebens des Vorratsgefäßes (93) in den Adapter (91) die Membran (94) durchtrennt.

9. Sterilisiereinrichtung nach einem der Ansprüche 1 - 8, **dadurch gekennzeichnet**, daß die Nadel eine Hohlkanüle (62) ist.

10. Sterilisiereinrichtung nach den Ansprüchen 8 und 9, **dadurch gekennzeichnet**, daß die Membran aus einem rißfest punktierbaren Material besteht.

## Claims

1. Sterilising device (16, 70, 80, 90) for an endoscope passage to which a vacuum may be applied with an adaptor (17, 71, 91) which may be inserted in a vacuum-tight manner into a free end of the endoscope passage and in which a through passage is formed extending from the endoscope passage to the exterior, and with a supply vessel (20) in which a liquid sterilising agent, which is vaporisable or atomisable under vacuum, is contained and which has all end region carrying an opening (19) with which the supply vessel may be inserted in a vacuum-tight manner into the through passage in the adaptor, whereby provided in the supply vessel there is a membrane (21, 44, 51, 61, 74, 84, 94) which is constructed to be destructable and which encloses the sterilising agent in the supply vessel, characterised in that the sterilising device (16, 70, 80, 90) has a needle (23, 45 52, 62, 76, 81, 95) whose point is directed from the exterior towards the membrane (21, 44, 51, 61, 74, 84, 94) and that the membrane (21, 44, 51, 61,74, 84, 94) and the needle (23, 45, 52, 62, 76, 81, 95) are constructed to be movable relative to one another such that in the course of the relative movement the tip of the needle (23, 45, 52, 62, 76, 81, 95) may puncture the membrane (21, 44, 51, 61, 74, 84, 94).

2. Sterilising device as claimed in claim 1, characterised in that the needle (23, 45, 52, 62, 76, 81, 95) is fixedly disposed in the sterilising device (16, 70, 80, 90) and the membrane (21, 44, 51, 61, 74, 84, 94) is constructed to be movable with respect to the needle (23, 45, 52, 62, 76, 81, 95), whereby the tip of the needle (23, 45, 52, 62, 76, 81, 95), whereby the tip of the needle (23, 45, 52, 62, 76, 81, 95) is directed particularly towards a central region of the membrane (21, 44, 51, 61, 74, 84, 94).

3. Sterilising device as claimed in one of claims 1 or 2, characterised in that the supply vessel (20, 40, 50, 60, 73, 83) and the adaptor (17, 71, 82) are fixedly connectable together in a defined position and the membrane (21, 44, 51, 61, 74, 84) is constructed of flexible material and so arranged at a distance from the tip of the needle (23, 45, 52, 62, 76, 81) that it may be sucked over the tip of the needle (23, 45, 52, 62, 76, 81) and may thus be punctured after the application of the vacuum to the endoscope passage (11).

4. Sterilising device as claimed in one of claims 1 - 3, characterised in that the membrane (21, 44, 51, 61, 74, 84, 94) comprises material which tears when damaged.

5. Sterilising device as claimed in one of claims 1 to 4, characterised in that the supply vessel (40) is of two-part construction in the transverse direction and the membrane (44) is secured with its edge (43) in the connection region of the two parts (41, 42).

6. Sterilising device as claimed in one of claims 1 to 5, characterised in that the adaptor (71, 82, 91) carries the needle (76, 81, 95).

7. Sterilising device as claimed in claim 6, characterised in that the membrane (74, 94) is disposed on the edge of the supply vessel (73, 93).

8. Sterilising device in which the needle (95) is constructed in the adaptor (91) as claimed in one of claims 6 or 7, characterised in that the adaptor (91) and the supply vessel (93) are constructed for vacuum-tight sliding of the supply vessel (93) within the adaptor (91) and the positions of the membrane (94) and the needle (95) are so selected that the tip of the needle (95) cuts through the membrane (94) during the sliding of the supply vessel (93) into the adaptor (91).

9. Sterilising device as claimed in one of claims 1 - 8, characterised in that the needle is a hollow cannula (62).

10. Sterilising device as claimed in claims 8 and 9, characterised in that the membrane comprises a tear-resistant puncturable material.

## Revendications

1. Dispositif de stérilisation (16, 70, 80, 90) pour un canal d'endoscope pouvant être évacué, muni d'un adaptateur (17, 71, 91) pouvant être inséré de façon étanche au vide dans une extrémité libre du canal de l'endoscope et comportant une perforation s'étendant du canal de l'endoscope vers l'extérieur, et d'un réservoir (20) contenant un agent de stérilisation liquide pouvant être vaporisés ou atomisé par application brusque d'un vide et comportant une partie d'extrémité munie d'une ouverture (10) avec laquelle le réservoir peut être inséré de façon étanche au vide dans la perforation de l'adaptateur, le réservoir étant muni d'une membrane (21, 44, 51, 61, 74, 84, 94) conformée de façon à être destructible et enfermant l'agent de stérilisation dans le réservoir, **caractérisé en ce que** le dispositif de stérilisation (16, 70, 80, 90) présente une aiguille (23, 45, 52, 62, 76, 81, 95) dont la pointe est dirigée de l'extérieur vers la membrane (21, 44, 51, 61, 74, 84, 94), la membrane (21, 44, 51, 61, 74, 84, 94) et l'aiguille (23, 45, 52, 62, 76, 81, 95) étant mobiles l'une par rapport à l'autre de façon à ce qu'au cours de ce mouvement relatif la pointe de l'aiguille (23, 45, 52, 62, 76, 81, 95) puisse percer la membrane (21, 44, 51, 61, 74, 84, 94).

2. Dispositif de stérilisation selon la revendication 1, **caractérisé en ce que** l'aiguille (23, 45, 52, 62, 76, 81, 95) est logée de façon fixe dans le dispositif de stérilisation (16, 70, 80, 90) alors que la membrane (21, 44, 51, 61, 74, 84, 94) est mobile par rapport à l'aiguille (23, 45, 52, 62, 76, 81, 95), la pointe de l'aiguille (23, 45, 52, 62, 76, 81, 95) étant en particulier dirigée vers une zone centrale de la membrane (21, 44, 51, 61, 74, 84, 94).

3. Dispositif de stérilisation selon l'une des revendications 1 ou 2, **caractérisé en ce que** le réservoir (20, 40, 50, 60, 73, 83) et l'adaptateur (17, 71, 82) peuvent être reliés l'un à l'autre en une position fixe définie, la membrane (21, 44, 51, 61, 74, 84) étant réalisée en un matériau flexible et se trouvant à une distance telle de la pointe de l'aiguille (23, 45, 52, 62, 76, 81) qu'elle puisse, lors de l'évacuation du canal (11) de l'endoscope, être aspirée contre la pointe de l'aiguille (23, 45, 52, 62, 76, 81) et être percée par celle-ci.

4. Dispositif de stérilisation selon l'une des revendications 1 - 3, **caractérisé en ce que** la membrane (21, 44, 51, 61, 74, 84) est constituée d'un matériau qui se déchire en cas de lésion.

5. Dispositif de stérilisation selon l'une des revendications 1 à 4, **caractérise' en ce que** le réservoir (40) est divisé transversalement, la membrane (44) étant fixée par son bord (43) dans la zone de raccordement des deux parties (41,42).

6. Dispositif de stérilisation selon l'une des revendications 1 à 5, **caractérisé en ce que** l'adaptateur (71, 82, 91) porte l'aiguille (76, 81, 95).

7. Dispositif de stérilisation selon la revendication 6, **caractérisé en ce que** la membrane (74, 94) est placée sur le bord du réservoir (73, 93).

8. Dispositif de stérilisation dont l'aiguille (95) est, conformément à l'une des revendications 6 ou 7, intégrée à l'adaptateur (91), **caractérisé en ce que** l'adaptateur (91) et le réservoir (93) sont conformés de façon à permettre un déplacement étanche au vide du réservoir (93) dans l'adaptateur (91), les positions de la membrane (94) et de l'aiguille (95) étant choisies de façon à ce que la pointe de l'aiguille (95) déchire la membrane (94) pendant l'introduction du réservoir (93) dans l'adaptateur (91).

9. Dispositif de stérilisation selon l'une des revendications 1 - 8, **caractérisé en ce que** l'aiguille est une canule creuse (62).

10. Dispositif de stérilisation selon les revendications 8 et 9, **caractérisé en ce que** la membrane est constituée d'un matériau indéchirable lors de la ponction.
